# EUROPÄISCHE PATENTSCHRIFT

(11) **EP 2 590 719 B1**
(45) Veröffentlichungstag und Bekanntmachung des Hinweises auf die Patenterteilung: **23.03.2016**
(21) Anmeldenummer: 11726382.2
(22) Anmeldetag: 08.06.2011
(51) Int. Cl.: B01D 3/00, B01D 5/00, B01D 3/14

(54) **VERFAHREN ZUR DESTILLATION VON TEMPERATUREMPFINDLICHEN FLÜSSIGKEITEN**
METHOD FOR DISTILLING TEMPERATURE-SENSITIVE LIQUIDS
PROCÉDÉ DE DISTILLATION DE LIQUIDES SENSIBLES À LA TEMPÉRATURE

(30) Priorität: 11.07.2010 DE 102010026835
(43) Veröffentlichungstag der Anmeldung: 15.05.2013
(73) Patentinhaber: Air Liquide Global E&C Solutions Germany GmbH, 60439 Frankfurt am Main (DE)
(72) Erfinder: BAUER, Jochen, 60487 Frankfurt am Main (DE); CASTILLO-WELTER, Frank, 61381 Friedrichsdorf (DE); KIRSTEN, Klaus, 55130 Mainz (DE); KREICH, Markus, 64853 Otzberg (DE); STEDEN, Christoph, 61440 Oberursel (DE); WALTER, Dominic, 64289 Darmstadt (DE); ZEYEN, Rudolf, 60435 Frankfurt am Main (DE)
(74) Vertreter: Keil & Schaafhausen Patent- und Rechtsanwälte PartGmbB
(86) Internationale Anmeldenummer: PCT/EP2011/059520
(87) Internationale Veröffentlichungsnummer: WO 2012/007233

(56) Entgegenhaltungen:
- EP-A1- 1 475 364
- DE-A1- 4 300 131
- DE-A1- 19 830 163
- DE-A1-102007 031 766
- DE-C- 824 787
- DE-C1- 10 207 460
- US-A- 4 365 081
- US-A- 4 404 062
- US-B1- 6 596 129
- US-B1- 6 883 788

## Beschreibung

Die Erfindung betrifft ein Verfahren zur Destillation von temperaturempfindlichen Flüssigkeiten, insbesondere von Acrylsäure und deren Estern, wobei die Flüssigkeit in einer Kolonne erhitzt und wenigstens teilweise verdampft wird, wobei der Dampf durch einen innerhalb der Kolonne vorgesehenen Kondensator geführt wird, in dem der Dampf wenigstens teilweise kondensiert wird und wobei die kondensierte Flüssigkeit wenigstens teilweise aus der Kolonne abgezogen wird.

Als Destillation bezeichnet man ein thermisches Trennverfahren, das dazu dient, ein Gemisch verschiedener, ineinander lösliche Stoffe zu trennen. Dabei wird zunächst das Ausgangsgemisch zum Sieden gebracht. Der entstehende Dampf, der sich aus den verschiedenen Komponenten der zu trennenden Lösung zusammensetzt, wird in einem Kondensator kondensiert und anschließend wird das flüssige Kondensat aufgefangen. Die Trennwirkung beruht auf der unterschiedlichen Zusammensetzung der siedenden Flüssigkeit und des gasförmigen Dampfes, wozu unterschiedliche Siedepunkte der zu trennenden Komponenten notwendig sind.

Als temperaturempfindliche Stoffe sind diejenigen Verbindungen anzusehen, die bereits bei Überschreitung ihres Siedepunktes um 10 bis 50 °C dazu neigen, sich zu zersetzen und/oder zu polymerisieren. Ein Beispiel für eine solche temperaturempfindliche Substanz ist Acrylsäure (C₃H₄O₂), die dazu neigt, explosionsartig zu polymerisieren (Polymerisationsenthalpie = 75 kJ/mol). Acrylsäure wird daher für ihre Lagerung bei Temperaturen von etwa 20°C zuvor mit Inhibitor versetzt, um die Polymerisationsrate zu limitieren. Bei der Destillation von Acrylsäure werden in den Kolonnensümpfen üblicherweise Temperaturen unterhalb von 100 °C bei gleichzeitig kurzen Verweilzeiten angestrebt.

Die US 4,404,062 beschreibt eine Kolonne mit mehreren außerhalb der Kolonne angeordneten Kondensatoren, wobei mindestens einer der zuerst durchströmten Kondensatoren als Rückflusskondensator ausgestaltet ist. Nur das Kondensat des zuletzt durchströmten Kondensators wird als Produkt abgeführt, weshalb das Produkt eine hohe Reinheit aufweist.

Die EP 1 097 742 A1 beschreibt ein Verfahren zur Destillation von Acrylsäure, bei dem die in der Destillationskolonne entstehenden Dämpfe vom Kolonnenkopf in einen außen liegenden Kondensator, üblicherweise ein Rohrbündelkondensator, geführt werden, in dem ihr Hauptteil kondensiert wird. Die Leichtsieder, also Komponenten mit einem niedrigeren Siedepunkt als Acrylsäure, werden in einem Nachkondensator auskondensiert, wobei die Kondensatoren mit Kühlwasser im Temperaturbereich zwischen 30 und 50 °C betrieben werden.

Die EP 1 475 364 A1 handelt ebenfalls von der Destillation von Acrylsäure oder deren Estern und beschreibt speziell, wie bereits in die Destillationskolonne ein Polymerisationsinhibitor zugegeben und somit eine Polymerisation in der Kolonne wirkungsvoll verhindert werden kann. Auch hier ist der Kondensator außerhalb der Kolonne vorgesehen.

Auch die US 6,596,129 B1 beschreibt eine Kolonne mit außenliegenden Kondensatoren.

Eine Kolonne mit außenliegendem Kondensator ist teuer, besonders wenn ein Rohrbündelkondensator eingesetzt wird. Kostenintensiv sind solche Verfahre vor allem dann, wenn mit einem hohen Vakuum destilliert wird. Die hohen Kosten ergeben sich dadurch, dass die Dampfleitung zwischen Kolonne und Kondensator wegen des geringen Drucks (auf Grund des hohen Vakuums) und der deshalb geringen Gasdichte (und also sehr hohen Gasgeschwindigkeiten) einen sehr großen Durchmesser aufweisen muss. Typisch sind hier Rohrleitungen mit einem Durchmesser im Bereich zwischen 0,8 und 2 m, wobei diese Dimensionen sehr stark von der Kapazität der Anlage sowie der jeweiligen Position der Kolonne im Gesamtverbund der Anlage und damit verknüpft dem benötigten Vakuum abhängen. Bei einer relativ großen Anlage wären die konventionellen Kopfdämpfe-Leitungen von Kolonnen mit tiefem Vakuum (Kolonnen mit hoher Acrylsäurekonzentration im Sumpf) in der Größenordnung von 1,5 - 2m. Diese Rohrleitungen erfordern hohen Aufwand zur Unterstützung und einen entsprechend aufwendigen Stahlbau.

Die Brüdenleitung zwischen Kolonne und Kondensator muss zudem bei einem außerhalb der Kolonne vorgesehenen Kondensator beheizt oder zumindest sehr gut isoliert werden, damit keine Acrylsäure kondensiert, die polymerisieren kann und über die Zeit Schichten aufbaut, welche den Querschnitt der Leitung verringern. In manchen Anwendungen muss diese Brüdenleitung auch zusätzlich mit Polymerisationsinhibitor benetzt werden.

Die technische Anforderung, in der Brüdenleitung einen möglichst geringen Druckverlust zu erzeugen, verlangt möglichst kurze Leitungen und damit eine Anordnung des Kondensators in direkter Nähe der Kolonne, bevorzugt unterhalb deren Dampf-Austrittsöffnung.

Das Kondensat wird bei außenliegendem Kondensator unterhalb des Kondensators in einem Behälter gesammelt und von dort zum Teil als Rücklauf auf den Trennteil der Kolonne gegeben. Die Rückspeisung erfolgt oberhalb des Sammelbehälters, so dass eine zusätzliche Pumpe erforderlich ist.

Die Integration des Kondensators in die Kolonne würde es erlauben, auf den externen Behälter und den dazu nötigen Stahlbau zu verzichten, da der Behälter als Auffangwanne unterhalb des Kondensators integriert wird. Kondensator und Sammelbehälter bilden eine Einheit, die in die Kolonne integriert wird..

Die integrierte Anordnung erlaubt es, den Rückfluss zu den Trennstufen der Kolonne mittels Schwerkraft (ohne Pumpe) sicherzustellen.

Aus der EP 0 839 896 B1 ist eine solche Integration des Kondensator in die Kolonne für das Raffinieren von essbaren Ölen bekannt. Durch die Destillation sollen hier unerwünschte freie Fettsäuren entfernt werden, ohne das in dem Öl enthaltene Carotin zu zerstören. Das Rohöl oder vorbehandelte Öl wird bei einem Druck von < 6 Pa und einer Temperatur von 160 bis 200 °C destilliert und die enthaltenen freien Fettsäuren im Öl durch Kondensation innerhalb des Destillierers entfernt. Zum Entfernen der freien Fettsäuren wird die Mischung aus Öl und freien Fettsäuren einem internen Kondensator ausgesetzt, der in einem Temperaturbereich zwischen dem Schmelzpunkt der freien Fettsäuren und einer Temperatur unterhalb des Kondensationspunktes arbeitet.

Ein in die Kolonne integrierter und von unten angeströmter Kondensator ist preiswerter als der außenliegende Kondensator, hat aber den Nachteil des höheren Druckverlustes.

Da sich hier Dampf und Flüssigkeit im Gegenstrom bewegen, kann es zudem bei hoher Belastung der Kolonne leicht zum teilweise oder vollständigen "Fluten" des Wärmetauschers kommen, bei dem das Ablaufen des Kondensats durch den aufwärts strömenden Dampf gehindert wird. Dies ist besonders kritisch, wenn es sich um ein temperaturempfindliches Kondensat handelt, das sich aufgrund der längeren Verweilzeit an der Oberfläche des Kondensators zersetzt und/oder polymerisiert.

Man versucht dieses Problem technisch zu beheben, indem man für den Kondensator Rohre mit größerem Durchmesser einsetzt. Als Konsequenz aus dieser Maßnahme resultieren jedoch auch geringere Geschwindigkeiten und Turbulenzen der Dämpfe im Inneren des Kondensators und daraus ein schlechterer Wärmeübergangskoeffizient. Dies erfordert wiederum eine größere spezifische Wärmetauscherfläche und damit verbundene höhere Kosten und größere Abmessungen.

Der Druckverlust ist somit sowohl bei einem innen- als auch bei einem außenliegenden Kondensator ein sehr wichtiges Kriterium, welches bei der Auslegung zu berücksichtigen ist. Dies wirkt sich insbesondere dann aus, wenn die Destillation bei verringertem Druck durchgeführt wird, wie es der Fall ist, wenn die Siedetemperatur der Komponenten gesenkt werden soll, um eine möglichst niedrige thermische Belastung der Produkte zu erreichen. Dies hängt damit zusammen, dass der Druck im Verdampfer bzw. Kolonnensumpf immer höher als der Druck im Kolonnenkopf bzw. in den Vakuumleitungen ist, und zwar um die Summe der Druckverluste von Kolonne und Kondensator. Höhere Druckverluste des Kondensators erzwingen also die Erzeugung eines höheren Vakuums. Die dazu nötige Ausrüstung wird mit zunehmender Leistung teurer und wartungsintensiver.

Zudem werden die Vakuumleitungen zwischen dem Kondensator und dem Vakuumerzeuger teurer, weil der verringerte Druck höhere Gasgeschwindigkeiten verursacht, die mit größeren Leitungsquerschnitten ausgeglichen werden müssen. Größere Leitungsquerschnitte führen zu Mehrkosten für die Leitung selbst als auch für deren Unterstützung und den Stahlbau.

Die Kondensation der Brüdendämpfe wird mit höherem Vakuum ebenfalls schwieriger. Mit sinkendem Druck im Kondensator verringert sich die Differenz zwischen Kühlwassertemperatur und Kondensationstemperatur der Dämpfe. Dem kann zum einen mit einer technisch aufwändigen und daher teuren Absenkung der Kühlwassertemperatur oder einer ebenfalls aufwändigen und teuren Vergrößerung der spezifischen Wärmetauscherfläche des Kondensators entgegengewirkt werden.

Die US 6,883,788 B1 offenbart eine Destillationskolonne mit einem ersten Wärmetauscher sowie einem zweiten Wärmetauscher. Der erste Kondensator wird in sämtlichen Ausführungsbeispielen so betrieben, dass der Dampf sowie die kondensierte Flüssigkeit im Gegenstrom durch den Kondensator geführt werden. Wird die Gasphase absteigend durch den Kondensator geführt, kommt es im nachgeschalteten Kondensator zu einer Führung der kondensierten Flüssigkeit im Gleichstrom zu dem Dampf.

Die DE 198 30 163 A1 beschreibt einen Kolonnenkopfkondensator mit einem ziehbaren Wärmetauschersystem. Der Kondensator ist an dem Deckel der Kolonne derart angebracht, dass der Kondensator herausgezogen und so einfach ausgetauscht werden kann. Die Dämpfe steigen an einer Trennwand entlang in den Kopf der Kolonne und das Kondensat wird im Gleichstrom zu den Dämpfen gebildet.

Sowohl die DE 43 00 131 A1 als auch die DE 10 2007 031 766 A1 beschreiben eine Kolonne mit Wärmetauschern, welche im Kolonnenkopf angebracht und so angeordnet werden, dass die kondensierenden Dämpfe und das Kondensat sich in eine Richtung bewegen. Unterhalb der Wärmetauscher werden Auffangbehälter für das Kondensat vorgesehen, welches aus der Kolonne entfernt wird und anschließend zum Teil in die Kolonne zurückgeführt wird. Das Verhältnis zwischen dem in die Kolonne zurückgeführten und dem abgeführten Teil des Kondensats wird über ein Regelventil gesteuert. Dabei erfordert die Abführung des Kondensats aus der Kolonne heraus eine ausreichende Isolation der Durchflussrohre, da sonst durch das rückgeführte Kondensat hohe Energieverluste erzeugt werden. Außerdem wird durch das komplexe Rohrsystem eine Auskristallisation von zur Kristallbildung neigenden Substanzen, bspw. Acrylsäure und deren Ester, begünstigt, da sich Kristallisationskeime bevorzugt an Unebenheiten, bspw. Rohrstückübergängen, ausbilden.

Es ist daher Aufgabe der vorliegenden Erfindung, die Destillation temperaturempfindlicher Flüssigkeiten ohne apparativen Mehraufwand bei gleichzeitig niedrigem Druckverlust zu ermöglichen.

Diese Aufgabe wird mit der Erfindung durch ein Verfahren mit den Merkmalen des Anspruchs 1 gelöst.

Dazu werden in der Destillationskolonne oberhalb der Kolonneneinbauten mehrere Pakete an Wärmetauschern, bevorzugt Plattenwärmetauschern, installiert. Die Dämpfe, die den Kopf der Kolonne erreichen, strömen zunächst nahezu ungehindert am Kondensatorpaket vorbei und werden von oben, also dem Kopf der Kolonne, in diesen zuerst durchströmten Kondensator eingeleitet. Auf dessen gekühlter Oberfläche kondensiert Flüssigkeit, die aufgrund der Schwerkraft nach unten Richtung Sumpf der Kolonne abfließt, sich also in der gleichen Richtung wie der noch nicht kondensierte Dampf bewegt, wodurch ein Flüssigkeitsstau sicher verhindert wird. Selbst hohe Brüdenbelastungen, also große Massenströme an Dampf, führen so nicht zum Fluten des Kondensators. Der kondensierte Flüssigkeitsfilm wird teilweise sogar beschleunigt durch das Dampfvolumen in die Auffangwanne geschoben.

An diesem zuerst durchströmten Kondensator wird bevorzugt der größte Anteil der kondensierbaren Dämpfe verflüssigt, da dies die Auslegung der Anlage vereinfacht.

Das Kondensat wird in einem Kondensatsammler gesammelt, der im Inneren der Kolonne angeordnet ist, wodurch das Kondensat nicht abgekühlt und ein entsprechend hoher apparativer Aufwand zur Isolierung von Rohren, die außerhalb der Kolonne verlaufen, vermieden wird. Ein Teil des gesammelten Kondensates wird aus der Kolonne abgeführt, ein anderer Teil wird erneut auf die Kolonne aufgegeben.

Das Temperaturprofil der Kolonne wird gemäß der Erfindung dadurch geregelt, dass der Anteil des entnommenen Kondensats als Stellgröße dient. Die Position des Kondensatsammlers im Inneren der Kolonne und möglichst oberhalb anderer Kolonneneinbauten, wie Trennböden etc., ermöglichen es, hier nicht die zurückgespeiste Menge, sondern die entnommene Menge an Kondensat zu regeln. Üblicherweise wird vom flüssigen Kopfprodukt einer Kolonne, d.h. den vom Kondensator kondensierten Dämpfen, ein Teil als Rückfluss zurück auf die Kolonne gegeben, während der andere Teil nach außen abgeführt wird. Durch die Position des Kondensatsammlers oberhalb der Trennstufen, z.B. Destillationsböden kann der Rückfluss indirekt geregelt werden, indem die nach außen abgeführte Menge verändert wird, d.h. reduziert man die nach außen geführte Menge, erhöht sich der Rückfluss dementsprechend um die Menge, die nicht mehr nach außen geführt wird. Das nicht entnommene Kondensat fließt aus dem Kondensatsammler nach unten auf weitere Kolonneneinbauten, insbesondere die vorgesehenen Trenneinrichtungen, und regelt das Temperaturprofil der Kolonne in der gleichen Weise wie beim Stand der Technik, der auf einer Regelung des zurückgespeisten Kondensates beruht.

Beim Stand der Technik wird die gesamte Menge an kondensierten Dämpfen (gesamtes flüssiges Kopfprodukt) mit Hilfe einer Pumpe gefördert (in manchen Fällen werden zwei Pumpen eingesetzt: Rückflusspumpe +Abstoßpumpe). Der zur Kolonne einzustellende Rückfluss wird üblicherweise mengengeregelt über einen Regelventil eingestellt. Die abzuführende Menge ergibt sich aus der Gesamtmenge an Dämpfen, die kondensiert wurde und nicht als Rückfluss zur Kolonne gegeben wurde. Meistens erfolgt die Abgabemenge über einen Flüssigkeitsstandregler am Kondensatsammelbehälter, der den Stand konstant hält und damit die Überschussmenge, die nicht als Rückfluss benötigt wird, abführt. In oben beschriebener Anordnung braucht die Rückflussmenge nicht gepumpt werden, sondern kann über Schwerkraft aus der Auffangwanne zu den unterhalb liegenden Trennstufen der Kolonne geführt werden. Auf diese Weise wird die bei einem außenliegenden Kondensat-sammelbehälter notwendige Rückspeisepumpe eingespart. Diese Lösung ist besonders dann vorteilhaft, wenn die Größe des Kondensatsammlers so ausgelegt ist, dass ohne weitere Ventile und andere Regelvorrichtungen durch den Überlauf selbst die geeignete Menge an Kondensat in die Kolonne zurückgeführt wird. Selbstverständlich sind jedoch auch andere Möglichkeiten zur Regelung des Volumenstroms denkbar.

Um eine möglichst vollständige Kondensation der abzutrennenden Komponente zu erreichen, sieht eine Ausgestaltung der Erfindung vor, mehrere Kondensatoren hintereinander zu schalten, wobei der zweite und/oder weitere Kondensatoren sowohl im Gleich- als auch Gegenstrom betrieben werden können.

Besonders vorteilhaft ist es, wenn der erste Kondensator mit Kühlmittel, mit einer Temperatur von ca. 18 bis 40 °C, bevorzugt 25 bis 35 °C, durchströmt wird und der zweite Kondensator mit einem Kühlmittel mit einer Temperatur von ca. 1 bis 20 °C, bevorzugt 5 bis 15 °C, durchströmt wird. Das Kühlmittel ist bevorzugt Wasser, kann jedoch z. B. auch ein Wasser/Ethylenglykolgemisch sein.

Der zweite Kondensator kann sowohl im Gegenstrom als auch nach Umlenkung der Brüden erneut im Gleichstrom durchströmt werden. An der Oberfläche des nachgeschalteten Konden-sators oder der nachgeschalteten Kondensatoren kondensieren die restlichen Dämpfe der schwersiedenden Komponenten sowie ein höherer Anteil an leichter siedenden Komponenten als am zuerst durchströmten Kondensator. Das Kondensat des zweiten Kondensators wird deshalb vollständig abgeführt, ohne es erneut zur Kolonne zu leiten. Der Vorteil der Abtrennung der leichter siedenden Komponenten von dem Kondensat des ersten Kondensators besteht darin, dass diese Kondensate weniger geeignet sind, das Temperaturprofil zu regeln. Leichtsieder verhalten sich in einer Trennkolonne ähnlich wie inerte Gase, die Turbulenzen und erhöhte Gasströmungen verursachen und damit die Trenn-leistung einer Kolonne verschlechtern.

Es liegt jedoch auch im Rahmen der Erfindung, die Kondensatströme zweier, mehrerer oder aller Kondensatoren zu mischen und als Gemisch zurück zu speisen. Dies hat den Vorteil einer apparativ einfachen Gestaltung, da nur eine Auffangvorrichtung und ein Kondensatsammler benötigt werden.

Um eine Polymerisation von kondensierten Stoffen zu verhindern, ist es vorteilhaft, einen geeigneten Inhibitor in die Kolonne einzubringen. Vorteilhafterweise wird dieser Inhibitor oberhalb aller Kolonneneinbauten, also oberhalb des Kondensators, des Kondensatsammlers, der Trennvorrichtungen etc. eingebracht. Es empfiehlt sich besonders, den Inhibitor von oben durch Versprühen oder Verdüsen auf die Oberflächen des Kondensators oder mehrerer Kondensatoren aufzubringen, da sich hier Kristalle und als Folge daraus mögliche Polymerisationskeime bilden können.

Eine Anlage zur Destillation von temperaturempfindlichen Flüssigkeiten, insbesondere von Acrylsäure und deren Estern, die zur Durchführung des erfindungsgemäßen Verfahrens geeignet ist umfasst eine Kolonne, in deren Innerem wenigstens ein Kondensator angeordnet ist, wobei die Kolonne und/oder der zuerst durchströmte Kondensator so aufgebaut ist, dass in dem zuerst durchströmten Kondensator der noch nicht kondensierte Dampf im Gleichstrom zu der kondensierten Flüssigkeit geführt wird. Die Führung des Dampfes im Gleichstrom wird vorteilhafterweise dadurch erreicht, dass der Eintritt des Dampfes in nach unten gerichtete Kondensatoröffnungen verhindert wird, so dass der Dampf zuerst am Kondensator vorbei weiter nach oben in den Kopf der Kolonne strömt und dort in Kondensatoröffnungen umgelenkt wird, so dass die Strömungsrichtung nun vom Kopf zum Sumpf der Kolonne umgelenkt wird. Durch die Schwerkraft läuft das Kondensat ebenfalls vom Kopf zum Sumpf, so dass Dampf und Kondensat im Gleichstrom geführt werden. Die Umlenkung des Kondensates kann apparativ durch eine Vielzahl von Möglichkeiten erfolgen. So sind zum Beispiel geeignete Ventile an den zum Sumpf hin ausgerichteten Öffnungen des Kondensators denkbar. Bevorzugt erfolgt die Umlenkung des Kondensators jedoch dadurch, dass die Auffangvorrichtung für das Kondensat so ausgelegt ist, dass sie die Öffnungen des Kondensators, die zum Sumpf hin ausgerichtet sind, abschirmt. Kondensator und Auffangwanne bilden üblicherweise eine Einheit, die nach unten geschlossen ist, so dass keine Dämpfe von unten, sondern bevorzugt von oben bzw. eventuell teilweise auch von der Seite am oberen Ende des Kondensators einströmen.

Eine vorteilhafte Ausgestaltung der Anlage sieht weiterhin vor, dass im Inneren der Kolonne ein Kondensatsammler angeordnet ist, wodurch das Kondensat automatisch die an dieser Position in der Kolonne herrschende Temperatur aufweist. Besonders günstig ist es, wenn die Auffangvorrichtung so dimensioniert wird, dass sie auch als Kondensatsammler dient.

Gemäß einer bevorzugten Ausgestaltung der Anlage ist mindestens ein, vorzugsweise alle Kondensator(en) ein Plattenkondensator, der eine apparativ einfach ausführbare und vergleichsweise billige Vorrichtung mit gleichzeitig großer Wärmeaustauschfläche ist.

Weiterbildungen, Vorteile und Anwendungsmöglichkeiten der Erfindung ergeben sich auch aus der nachfolgenden Beschreibung von Ausführungsbeispielen und der Zeichnung. Dabei bilden alle beschriebenen und/oder bildlich dargestellten Merkmale für sich oder in beliebiger Kombination den Gegenstand der Erfindung unabhängig von ihrer Zusammenfassung in den Ansprüchen oder deren Rückbeziehung.

Es zeigen
- Fig. 1: das Schema einer erfindungsgemäßen Destillationskolonne mit zwei innenliegenden Kondensatoren, wobei beide Kondensatoren im Gleichstrom betrieben werden;
- Fig. 2: das Schema einer erfindungsgemäßen Destillationskolonne mit zwei innenliegenden Kondensatoren, wobei der erste Kondensator im Gleich- und der zweite im Gegenstrom betrieben wird;
- Fig. 3: das Schema einer erfindungsgemäßen Destillationskolonne mit zwei innenliegenden Kondensatoren, wobei der erste Kondensator im Gleich- und der zweite im Gegenstrom betrieben wird und beide Kondensatoren eine gemeinsame Auffangvorrichtung aufweisen;
- Fig. 4: das Schema einer erfindungsgemäßen Destillationskolonne mit drei innenliegenden Kondensatoren, wobei der erste und der zweite Kondensator im Gleich- und der dritte im Gegenstrom betrieben wird.

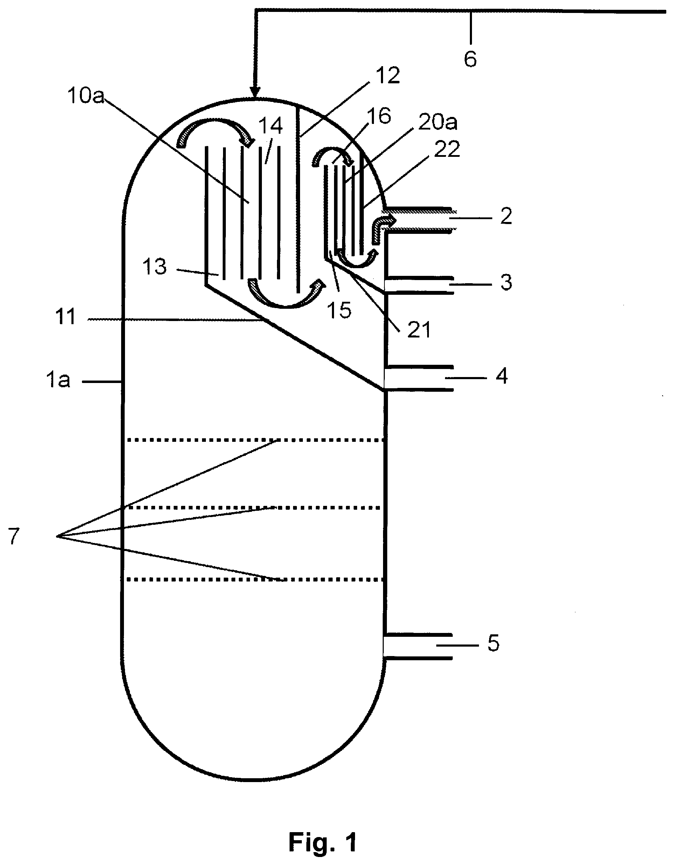

In Fig. 1 ist eine erfindungsgemäße Kolonne 1 a dargestellt, die am Kopfbereich, nämlich oben, zwei Kondensatoren 10a, 20a aufweist. Der in der Kolonne aufsteigende, erhitzte Dampf, insbesondere Acrylsäuredampf, wird über eine Ablaufplatte 11 so umgeleitet, dass er nicht in die nach unten zum Sumpf hin gerichteten Öffnungen 13 der Kondensatoren 10a, 20a eintreten kann, sondern am Kondensator 10a vorbeiströmt. Oberhalb des Kondensators 10a verhindert eine Umlenk- und Absperrvorrichtung 12, die zum Beispiel eine weitere Platte sein kann, dass der Dampf in den Kondensator 20a eintreten kann. Aufgrund der erzwungenen Strömung strömt, wie durch Pfeile angedeutet, der Dampf nun in die nach oben gerichteten Öffnungen 14 des Kondensators 10a ein. In diesem Kondensator 10a werden Teile des Dampfes auskondensiert. Das an den Wänden des Kondensators 10a niedergeschlagene Kondensat rinnt durch die Schwerkraft nach unten, wo es durch eine Auffangvorrichtung 11 zu einer Ableitung 4 von Kondensaten mit niedrigerem Leichtsiederanteil geführt wird.

Die noch gasförmigen Bestandteile strömen, wie durch Pfeile angedeutet, an der Umlenkvorrichtung 12 vorbei zum zweiten Kondensator 20. Wie schon beim zuerst durchströmten Kondensator 10a verhindert in der in Fig. 1 schematisch dargestellten Kolonne auch beim Kondensator 20a eine Vorrichtung zum Auffangen des Kondensates, dass der Dampf in die zum Sumpf hin ausgerichteten Öffnungen 15 des Kondensators 20a eintritt. Stattdessen lenkt eine Auffangvorrichtung 21 den Dampf entlang der Umlenkvorvorrichtung 12 an dem Kondensator 20a vorbei, so dass der Dampf in die nach oben ausgerichteten Öffnungen 16 des zweiten Kondensats 20a eintritt. Wie auch in dem zuerst durchströmten Kondensator 10a strömt nun der Dampf zusammen mit dem entstehenden Kondensat im Gleichstrom in Richtung des Sumpfes der Kolonne 1 a. Entstehendes Kondensat wird in der Auffangvorrichtung 21 aufgefangen und durch eine Ableitung 3 von Kondensaten mit höherem Leichtsiederanteil abgeführt. Verbleibender Dampf strömt an einer weiteren Umlenkvorrichtung 22 vorbei in eine Ableitung 2 der nicht kondensierten Bestandteile zum Vakuumsystem oder zur Rückführung in die Kolonne 1 a.

Leitung 5 steht stellvertretend für die Möglichkeit, weitere Komponenten aus dem Sumpf der Kolonne 1 a abzuziehen und/oder den Feed (Zufuhrstrom) oder Teile des Feeds der Kolonne 1a einzuspeisen. Dargestellt sind zudem schematisch in der Kolonne 1 a enthaltene Kolonneneinbauten 4, wie Flüssigkeitsverteiler, Trenn- und Sammelböden, Auflageroste, Niederhalteroste, Tropfenabscheider, Gasverteiler, Packungen, Füllkörperschüttungen und Sonderbauteile.

Leitung 6 stellt eine Leitung zur Zuführung eines Inhibitors zur Verhinderung von Polymerisation dar. Dieser Inhibitor kann zum Beispiel in ein nicht dargestelltes Verteilersystem am Kopf der Kolonne 1 a eingebracht werden und sich von dort in der Kolonne 1a verteilen. Da sich an jeder Oberfläche Kristallisationskeime bilden können, von denen eine Polymerisation ausgehen kann, ist es besonders günstig, den Polymerisationsinibitor direkt auf Einbauten, wie etwa die Kondensatoren 10a, 20a, zum Beispiel durch direktes Aufsprühen, zu verteilen. Alternativ kann am Kopf der Kolonne 1a auch eine Düse vorgesehen sein, die den Inhibitor vernebelt. Begünstigt ist auch hier vor allem eine möglichst vollständige Benetzung des zuerst durchströmten Kondensators 10a, da von dort Inhibitor durch den Dampf und die Flüssigkeit auf andere Bauteile der Kolonne 1 a geführt werden kann. Der Inhibitor kann in Abhängigkeit von der Zusammensetzung der zu kondensierenden Dämpfe sowohl allen Kondensatoren als auch nur einigen oder nur dem Hauptkondensator zugeführt werden.

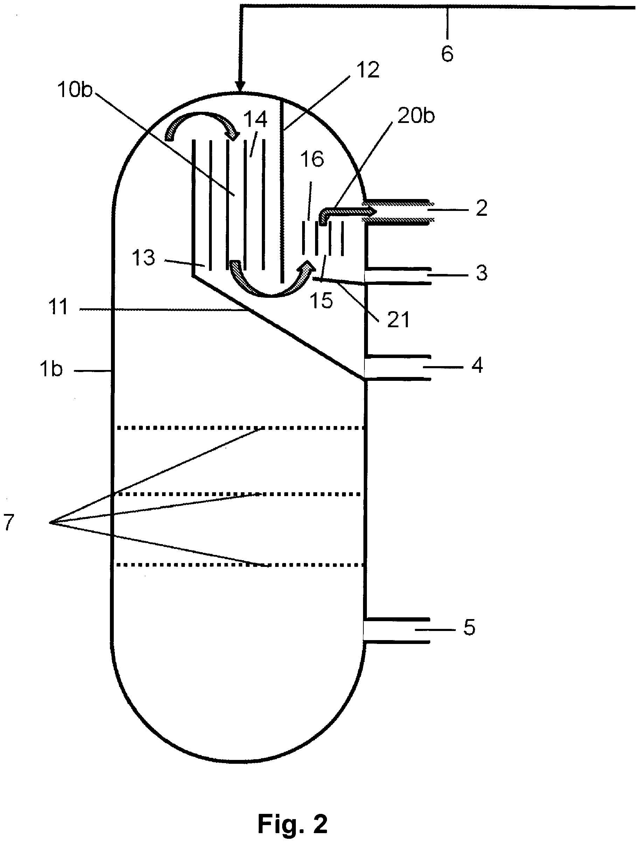

Fig. 2 zeigt eine Kolonne 1 b gemäß einer zweiten Ausführungsform mit zwei innenliegenden Kondensatoren 10b, 20b. Hier wird jedoch der Dampf nach Durchströmen des Kondensators 10b im Gleichstrom mit dem dort gewonnenen Kondensat nicht von der Auffangvorrichtung 21 am direkten Eintritt in den Kondensator 20b gehindert. Der Dampf strömt vielmehr, wie durch Pfeile angedeutet, vorbei an der Auffangvorrichtung 21, tritt in die nach unten, in Richtung des Sumpfes ausgerichteten Öffnungen 15 des Kondensators 20b ein und durchströmt diesen von unten nach oben. Entstehendes Kondensat fließt gleichzeitig vom Kopf zum Sumpf, so dass Kondensat und Dampf im Kondensator 20b im Gegenstrom geführt werden. Auch hier wird das Kondensat des zweiten Kondensators 20b in der separaten Auffangvorrichtung 21 gesammelt und durch die Ableitung 3 ausgeleitet. Verbleibender Dampf wird durch die Ableitung 2 ausgeleitet.

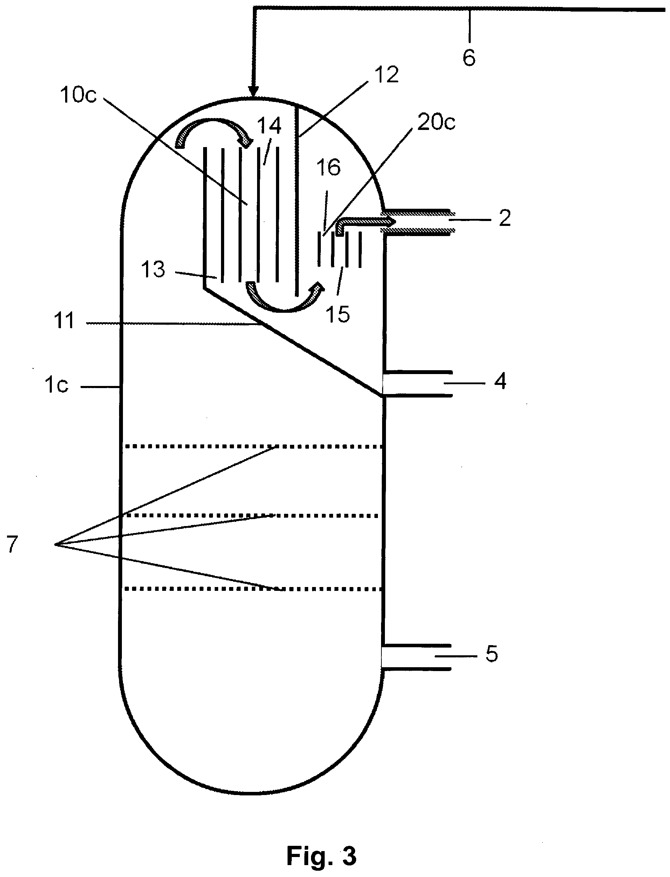

Bei der in Fig. 3 dargestellten dritten Ausführungsform wird der Dampf wie in Fig. 2 im zuerst durchströmten Kondensator 10c im Gleichstrom mit dem gewonnenen Kondensat und im zweiten Kondensator 20c im Gegenstrom zu dem gewonnenen Kondensat geführt. Bei dieser Ausführungsform ist auf eine zweite Einrichtung zum Sammeln des Kondensates, welches in Kondensator 20c anfällt, verzichtet worden. Kondensat, das im Kondensator 20c anfällt, tropft in die Auffangvorrichtung 11, in der auch das Kondensat aus dem zuerst durchströmten Kondensator 10c aufgefangen wird. Die Mischung der beiden Kondensatströme wird über die Ableitung 4 aus der Kolonne 1 c abgezogen. Der verbleibende Dampf strömt durch die Ableitung 2 ab.

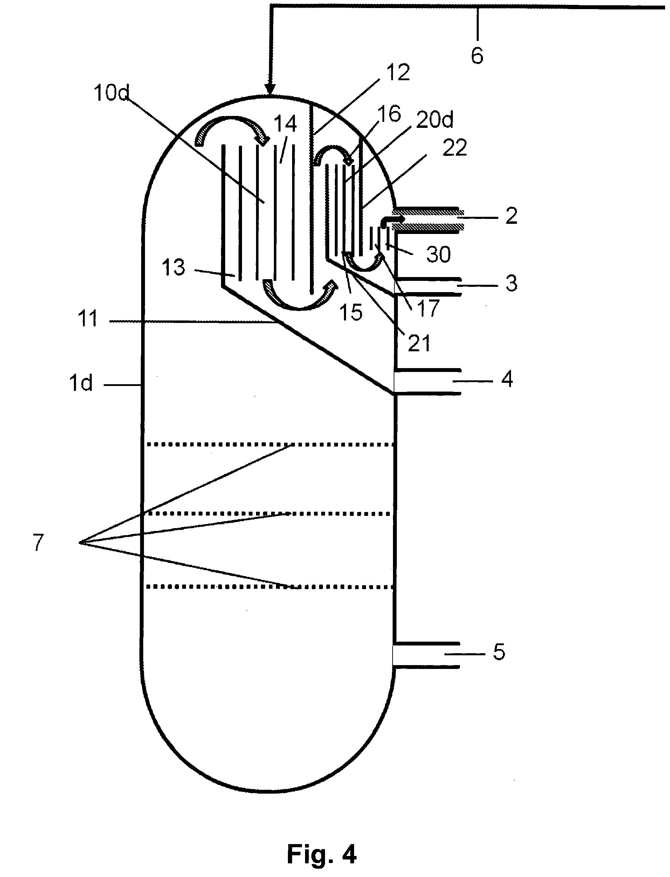

Bei der vierten Ausführungsform gemäß Fig. 4 sind drei im Inneren der Kolonne 1d liegende Kondensatoren 10d, 20d und 30 dargestellt. Der Dampf tritt dabei in den zuerst durchströmten Kondensator 10d so ein, dass er im Gleichstrom mit dem im Kondensator 10d anfallenden Kondensat geführt wird. Auch der zweite Kondensator 20d wird ähnlich wie bei der ersten Ausführungsform im Gleichstrom betrieben. Nach Austritt aus dem Kondensator 20d strömt, wie durch Pfeile angedeutet der noch vorhandene Dampf in die in Richtung Sumpf gerichteten Öffnungen 17 des dritten Kondensators 30 ein, so dass hier eine Führung im Gegenstrom zu dem flüssigen Kondensat des Kondensators 30 erfolgt. Grundsätzlich ist jedoch auch im dritten Kondensator 30 eine Führung des Dampfes im Gleichstrom möglich.

Hinsichtlich der Temperatur des verwendeten Kühlwassers kann der dritte Kondensator 30 ebenso wie der Kondensator 20d von dem gleichen Kühlwasser wie der zuerst durchströmte Kondensator 10d durchströmt werden. Der dritte Kondensator 30 kann auch mit Kühlwasser betrieben werden, welches die gleiche Temperatur wie das in Kondensator 20d verwendete Kühlwasser aufweist, oder der dritte Kondensator 30 wird mit einem Kühlwasser mit einer dritten Temperatur betrieben, wobei diese Temperatur bevorzugt zwischen oder unterhalb der Kühlwassertemperatur der beiden anderen Kondensatoren 10d und 20d liegt.

Die Kondensate der dritten und weiteren möglichen Kondensatoren können sowohl dem Kondensat des ersten Kondensators 10d als auch dem Kondensat des zweiten Kondensators 20d zugegeben werden oder über eine eigene Leitung abgeführt werden.

### Vergleichsbeispiel

In einer bestehenden Anlage zur Herstellung von 30.000 t Acrylsäure pro Jahr wird die Acrylsäure zur Reinigung destilliert. Bei der dazu verwendeten Destillationseinrichtung ist über dem Trennteil mit acht theoretischen Böden außerhalb der Kolonne ein Rohrbündelkondensator aufgesetzt, der von unten mit Dampf angeströmt wird. Diese Aufstellung entspricht im Wesentlichen dem in der EP 1 475 364 A1 beschriebenen außenliegenden Kondensator.

Dieser Rohrbündelkondensator verursacht unter normalen Betriebsbedingungen ein Druckverlust von 2,5 kPa. Der darunterliegende Trennteil der Kolonne verursacht bei normaler Betriebsweise der Anlage weitere 9,5 kPa Druckverlust, womit die gesamte Kolonne in Summe 12 kPa Druckverlust aufweist. Das installierte Vakuumsystem erzeugt bei normalen Produktionsraten einen absoluten Druck von 7 kPa. Bei dieser Ausführung, die dem Stand der Technik entspricht, stellt sich so ein Druck im Verdampfer von ca. 20 kPa und ein korrespondierender Siedepunkt des acrylsäurehaltigen Sumpfprodukts von ca. 96 °C ein.

Durch Austausch des Kopfteils der Kolonne kann die Kolonne erfindungsgemäß ausgestattet werden. Der geflanschte Rohrbündelwärmetauscher wird entfernt und ein Plattenkondensator gemäß der hier beschriebenen Erfindung wird im Inneren der Kolonne installiert. Dadurch fällt der Druckverlust des Kondensators von 2,5 kPa auf < 0,2 kPa. Allein durch diese Maßnahme und ohne den Trennteil der Kolonne oder das Vakuumsystem zu verändern, erreicht man so bei unveränderter Anlagenkapazität eine Druckabsenkung im Verdampfer um > 2 kPa. Damit fällt der absolute Druck im Verdampfer von ca. 20 kPa auf ca. 18 kPa. Als Folge des so verringerten Drucks verringert sich der Siedepunkt des Sumpfproduktes von ca. 96 °C auf < 93 °C.

Durch diese Verringerung der Sumpftemperatur kann nicht nur die Destillationskolonne energetisch effizienter betrieben werden, sondern es verringert sich auch die Polymerisationsneigung des Produktes und sein thermischer Abbau erkennbar: So nimmt die Menge an gebildetem Dimer aus der unerwünschten Nebenreaktion der Acrylsäure durch diese Maßnahme von ca. 110 kg/h auf < 75 kg/h ab; die Produktfarbe der enthaltenen Acrylsäure verbessert sich durch diese Maßnahme von ca. 8 auf ca. 7 Einheiten ("Hazen" Farbzahl nach APHA).

Die Installationskosten des Systems liegen mehr als 30 % unterhalb der aus dem Stand der Technik bekannten Varianten.

### Bezugszeichenliste

- 1 a-d: Kolonne
- 2: Leitung
- 3: Leitung
- 4: Leitung
- 5: Leitung
- 6: Leitung
- 7: Kolonneneinbauten
- 10a-d: Kondensator
- 11: Auffang- und Sammelvorrichtung des Kondensats
- 12: Trennvorrichtung
- 13-17: Kondensatoröffnungen
- 20a-d: Kondensator
- 21: Auffangvorrichtung des Kondensats
- 22: Trennvorrichtung
- 30: Kondensator

## Patentansprüche

1. Verfahren zur Destillation von temperaturempfindlichen Flüssigkeiten, nämlich von Acrylsäure und deren Estern, wobei die Flüssigkeit in einer Kolonne erhitzt und wenigstens teilweise verdampft wird, wobei der Dampf durch einen innerhalb der Kolonne vorgesehenen Kondensator geführt wird, in dem der Dampf wenigstens teilweise kondensiert wird und wobei die kondensierte Flüssigkeit wenigstens teilweise aus der Kolonne abgezogen wird, wobei der noch nicht kondensierte Dampf im Gleichstrom zu der kondensierten Flüssigkeit durch den zuerst durchströmten Kondensator geführt wird, **dadurch gekennzeichnet, dass** in der Kolonne wenigstens zwei hintereinander geschaltete Kondensatoren vorgesehen sind, die nacheinander durchströmt werden, dass der zweite Kondensator und/oder weitere Kondensatoren im Gleich- oder Gegenstrom betrieben werden, dass das Kondensat in einem, im Inneren der Kolonne angebrachten, Kondensatsammler gesammelt wird, wobei ein Teil des gesammelten Kondensats aus der Kolonne abgeführt und der andere Teil des gesammelten Kondensats erneut auf die Kolonne aufgegeben wird und wobei das Temperaturprofil der Kolonne durch den Anteil des abgeführten Kondensats geregelt wird.

2. Verfahren nach Anspruch 1, **dadurch gekennzeichnet, dass** der erste Kondensator mit Kühlmedium mit einer Temperatur von 18 bis 40 °C, bevorzugt 25 bis 35 °C, und mindestens ein weiterer Kondensator mit Kühlmedium mit einer Temperatur von 1 bis 20°C, bevorzugt 5 bis 15 °C, betrieben wird.

3. Verfahren nach Anspruch 1 oder 2, **dadurch gekennzeichnet, dass** das Kondensat des zweiten und/oder mindestens eines weiteren Kondensators getrennt vom Kondensat des ersten Kondensators gesammelt und ohne Rückleitung auf die Kolonne vollständig abgeführt wird.

4. Verfahren nach einem der Ansprüche 1 bis 3, **dadurch gekennzeichnet, dass** das Kondensat des zweiten Kondensators und/oder mindestens eines weiteren Kondensators zusammen mit Kondensat des ersten Kondensators abgeführt und als Gemisch zurück gespeist wird.

5. Verfahren nach einem der vorherigen Ansprüche, **dadurch gekennzeichnet, dass** ein Polymerisationsinhibitor in die Kolonne eingebracht wird.

## Claims

1. A process for the distillation of temperature-sensitive liquids, namely of acrylic acid and its esters, wherein the liquid is heated and at least partly evaporated in a column, wherein the vapor is guided through a condenser provided inside the column, in which the vapor is at least partly condensed, and wherein the condensed liquid is at least partly withdrawn from the column, wherein the vapor not condensed yet is guided through the condenser cocurrently to the condensed liquid
**characterized in that** in the column at least two series-connected condensers are provided, that the second condenser and/or further condensers are operated in cocurrent or countercurrent flow, that the condensate is collected in a condensate collector mounted in the interior of the column, that one part of the collected condensate is discharged from the column and the other part of the collected condensate is again charged to the column, and that the temperature profile of the column is controlled by the amount of condensate discharged.

2. The process according to claim 1, **characterized in that** the first condenser is operated with cooling medium with a temperature of 18 to 40 °C, preferably 25 to 35 °C, and at least one further condenser is operated with cooling medium with a temperature of 1 to 20 °C, preferably 5 to 15 °C.

3. The process according to claim 1 or 2, **characterized in that** the condensate of the second and/or at least one further condenser is collected separate from the condensate of the first condenser and is discharged completely without recirculation to the column.

4. The process according to any of claims 1 to 3, **characterized in that** the condensate of the second condenser and/or at least one further condenser is discharged together with condensate of the first condenser and fed back as mixture.

5. The process according to any of the preceding claims, **characterized in that** a polymerization inhibitor is introduced into the column.

## Revendications

1. Procédé destiné à distiller des liquides thermosensibles, à savoir de l'acide acrylique et ses esters, lors duquel on chauffe le liquide dans une colonne et on le fait évaporer au moins en partie, la vapeur étant conduite à travers un condensateur prévu à l'intérieur de la colonne dans lequel la vapeur se condense au moins en partie et le liquide condensé étant soutiré au moins en partie de la colonne, la vapeur non condensée étant conduite dans un flux identique à celui du liquide condensé à travers le condensateur d'abord irrigué, **caractérisé en ce que** dans la colonne sont prévus au moins deux condensateurs montés l'un derrière l'autre, qui sont irrigués successivement, **en ce que** le deuxième condensateur et ou des condensateurs supplémentaires sont exploités à flux identique ou à contre-flux, **en ce que** le produit de condensation est collecté dans un collecteur à condensats monté à l'intérieur de la colonne, une partie du produit de condensation collecté étant évacuée hors de la colonne et l'autre partie du produit de condensation collecté étant redistribuée dans la colonne et le profil de température de la colonne étant régulé par la part de produit de condensation évacué.

2. Procédé selon la revendication 1, **caractérisé en ce que** le premier condensateur est exploité avec un agent de refroidissement à une température de 18 à 40°C, de préférence de 25 à 35°C et au moins un condensateur supplémentaire est exploité avec un agent de refroidissement à une température de 1 à 20 °C, de préférence de 5 à 15 °C.

3. Procédé selon la revendication 2 ou la revendication 3, **caractérisé en ce que** le produit de condensation du deuxième condensateur ou de l'au moins un condensateur supplémentaire est collecté séparément du produit de condensation du premier condensateur et totalement évacué, sans retour vers la colonne.

4. Procédé selon l'une quelconque des revendications 1 à 3, **caractérisé en ce que** le produit de condensation du deuxième condensateur ou de l'au moins un condensateur supplémentaire est évacué en commun avec le produit de condensation du premier condensateur et réalimenté en tant que mélange.

5. Procédé selon l'une quelconque des revendications précédentes, **caractérisé en ce qu'**on introduit dans la colonne un inhibiteur de polymérisation.
